# EUROPEAN PATENT APPLICATION

(11) **EP 1 336 644 A1**
(43) Date of publication of application: **20.08.2003**
(21) Application number: 01978922.1
(22) Date of filing: 25.10.2001
(51) Int. Cl.: C08L 83/12, A61K 7/00, A61K 7/02

(54) **WATER-IN-OIL EMULSION COMPOSITION AND EMULSION COSMETICS MADE BY USING THE SAME**

(30) Priority: 26.10.2000 JP 2000327862; 01.02.2001 JP 2001025291
(71) Applicant: SHISEIDO COMPANY, LTD., Chuo-ku, Tokyo 104-8010 (JP)
(72) Inventor: SATO, Tomoko, SHISEIDO RESCH CENTER(SHIN-YOKOHAMA), Kanagawa 224-8558 (JP); WATANABE, Kei, SHISEIDO RESCH CENTER SHIN-YOKOHAMA, Kanagawa 224-8558 (JP); MATSUZAKI, Fumiaki, SHISEIDO RESCH CENTER SHiN-YOK, Kanagawa 224-8558 (JP); YANAKI, Toshio, SHISEIDO RSCH CENTER SHIN-YOKOHAMA, Kanagawa 224-8558 (JP)
(74) Representative: Quantin, Bruno
(86) International application number: JP0109411
(87) International publication number: WO02034834

(57) **Abstract**

A high internal aqueous phase water-in-oil type emulsion composition including (a) a high molecular weight polyether-modified silicone having a number average molecular weight of 30,000 or more, (b) a polyether-modified silicone having a number-average molecular weight of 10,000 or less, or a sucrose fatty acid ester and/or polyglyceryl fatty acid ester in the state of liquid at ordinary temperature and having an HLB of 4 or less, (c) a silicone-based oil and (d) an inorganic salt and/or organic salt, having a fresh, sharp feeling in use, and exhibiting an excellent emulsified state, as well as an emulsion cosmetic composition using the same.

## Description

### TECHNICAL FIELD

The present invention relates to a water-in-oil type emulsion composition and emulsion cosmetic composition. More particularly, it relates to a high internal aqueous phase water-in-oil type emulsion cosmetic having a fresh, sharp feeling in use and exhibiting an excellent emulsified state and an emulsion cosmetic composition using the same.

### BACKGROUND ART

In the past, in water-in-oil type emulsion compositions used for emulsion cosmetic compositions, etc., to obtain a fresh, sharp feeling in use, the practice has been to formulate a specific lyophilic sucrose fatty acid ester (see Japanese Unexamined Patent Publication (Kokai) No. 9-239259) or to formulate a polyether-modified silicone (see Japanese Unexamined Patent Publication (Kokai) No. 1-236936), as an emulsifier with a good stability of the base even having a high internal phase ratio.

However, in the case of an emulsion having a high internal aqueous phase ratio, the particles are closely packed together and contact each other, and therefore, tend to easily combine together and easily lose their emulsified state. Therefore, with only the above emulsifier, the long-term Stability of the water-in-oil type emulsion composition at a high temperature was insufficient.

Further, to make the feeling in use of the emulsion composition sharper, the practice has normally been to use a silicone oil as the oil in the emulsion composition. Therefore, it is necessary to select an emulsifier able to emulsify a silicone oil.

### SUMMARY OF THE INVENTION

Accordingly, objects of the present invention are to provide a high internal aqueous phase water-in-oil type emulsion composition having a fresh, sharp feeling in use and exhibiting an excellent emulsified state and an emulsion cosmetic composition using the same.

In accordance with the present invention, there is provided a water-in-oil type emulsion composition comprising:
(a) at least one high molecular weight polyether-modified silicone having a molecular weight of at least 30,000 and having the formula (I): 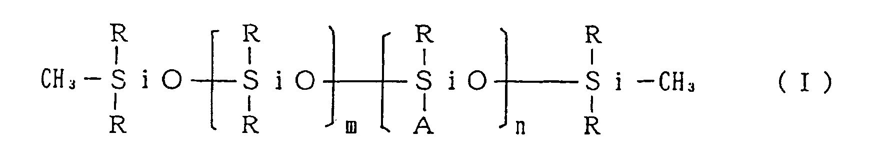 wherein A is a polyoxyalkylene group having the formula-C₃H₆O(C₂H₄O)ₐ(C₃H₆O)_{b}R', wherein R' is a group selected from the group consisting of a hydrogen atom, an acyl group, and a C₁ to C₄ alkyl group, a is an integer of 5 to 50, and b is an integer of 5 to 50, R is a methyl group or a phenyl group, m is an integer of 50 to 1000, and n is an integer of 1 to 40;
(b) at least one polyether-modified silicone having a molecular weight of 10,000 or less and having the formula (II): 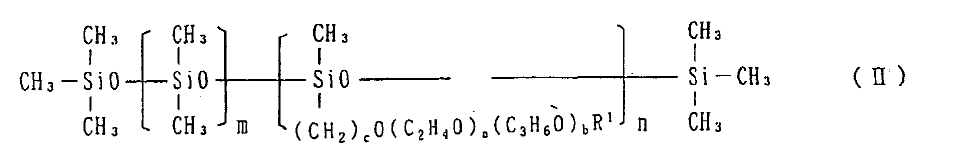 wherein R¹ indicates a hydrogen atom or a C₁ to C₅ alkyl group, m is, on average, a number of 1 to 150, n is, on average, a number of 1 to 50, a and b are independently, on average, numbers of 0 to 35 and c is a number of 1 to 5;
(c) a silicone-based oil; and
(d) at least one salt selected from the group consisting of inorganic salts and organic salts.

In accordance with the present invention, there is also provided a water-in-oil type emulsion composition where, in the above composition, the component (b) is at least one fatty acid ester selected from the group consisting of sucrose fatty acid esters and polyglyceryl fatty acid esters liquid at ordinary temperature and having an HLB value of 4 or less.

### MODE OF CARRYING OUT THE INVENTION

The configuration of the present invention will now be explained in detail.

The present inventors engaged in in-depth research considering the above problems and, as a result, found that by using a specific high molecular weight polyether-modified silicone, etc. having a surface activating capability, and a capability of gelling an external oil phase etc., and by including an inorganic salt or organic salt in the aqueous phase, it is possible to obtain a high internal aqueous phase water-in-oil emulsion composition containing a silicone oil in the oil phase ingredients, whereby the present invention was completed.

According to the present invention, it is possible to formulate an ultraviolet absorbing agent or an oil having a high polarity and capable of dissolving oil soluble chemicals, such as vitamins, which are difficult to formulate in a conventional water-in-oil type emulsion composition, if in an amount of 10% by weight or less.

The high molecular weight polyether-modified silicone (a) having a number-average molecular weight of 30,000 or more usable in the present invention is an organopolysiloxane having a polyoxyalkylene group shown in the above formula (I). For example, this is an emulsifier disclosed in Japanese Unexamined Patent Publication (Kokai) No. 5-311076.

Examples of the acyl group of the group R' in the formula (I) are specifically a formyl group, acetyl group, propionyl group, butyryl group, acryloyl group, benzoyl group, toluoyl group, etc. Examples of the C₁ to C₄ alkyl group are specifically a methyl group, ethyl group, isopropyl group, n-propyl group, t-butyl group, n-butyl group, etc.

The number-average molecular weight of the high molecular weight polyether-modified silicone (a) is 30,000 or more, preferably 50,000 or more, more preferably 50,000 to 200,000. This is because, if the molecular weight is less than 30,000, the emulsifiability with respect to nonpolar oils other than silicone oils is decreased.

The amount blended of the high molecular weight polyether-modified silicone able to be used in the present invention is not particularly limited, but preferably is 0.1 to 10.0% by weight, more preferably 0.3 to 5.0% by weight, based upon the total weight of the composition. If this formulated amount is less than 0.1% by weight, the function as an emulsifier is liable not to be sufficiently exhibited. Further, even if formulated in an amount of more than 10.0% by weight, the sticky feeling becomes unpreferably stronger.

The polyether-modified silicone (b) having a number-average molecular weight of 10,000 or less usable in the present invention is an organopolysiloxane having a polyoxyalkylene group shown in the above formula (II), and including for example, KF6017 (trade mark) made by Shin-etsu Chemical.

As the C₁ to C₅ alkyl group of the group R¹ in the formula (II), specifically a methyl group, ethyl group, isopropyl group, n-propyl group, t-butyl group, n-butyl group, n-pentyl group, isopentyl group, etc. may be illustrated.

If the molecular weight of the polyether-modified silicone is more than 10,000, the movability in a solution becomes lower, the silicone cannot be adsorbed on the surface of the emulsion particles immediately at the time of stirring and cannot work sufficiently as a low molecular weight active agent for preparing fine particles. The preferable number-average molecular weight is 3000 to 8000.

The amount formulated of the polyether-modified silicone (b) having a molecular weight of 10,000 or less used in the present invention is not particularly limited, but preferably is 0.05 to 10.0% by weight, more preferably 0.1 to 5.0% by weight, based upon the total weight of the composition. If the formulated amount is less than 0.05% by weight, the emulsifiability is liable not to be manifested, while even if formulated in an amount more than 10.0% by weight, no further improvement of the emulsion stability can be expected.

The sucrose fatty acid esters and the polyglyceryl fatty acid esters of the other component (b) usable in the present invention are preferably those having a C₈ to C₁₈ saturated fatty acid or C₁₆ to C₂₂ unsaturated fatty acid in an amount of at least 20% by weight of the fatty acid components.

The specific examples of the sucrose fatty acid esters are a sucrose caprylic acid ester, sucrose capric acid ester, sucrose lauric acid ester, sucrose myristic acid ester, sucrose palmitic acid ester, sucrose stearic acid ester, sucrose oleic acid ester, sucrose eruca acid ester, or sucrose isostearic acid ester. The ratio of the polyester including a diester (hereinafter abbreviated as "polyester") is preferably 100% by weight, but is at least 95% by weight, preferably at least 97% by weight is preferable.

The specific examples of the polyglyceryl fatty acid esters area a polyglyceryl caprylic acid ester, polyglyceryl capric acid ester, polyglyceryl lauric acid ester, polyglyceryl myristic acid ester, polyglyceryl stearic acid ester, polyglyceryl oleic acid ester, and polyglyceryl eruca acid ester. Further, as the degree of polymerization of the glycerol, 2 to 10 is preferable. In the present invention, it is possible to use, as the component (b), one or two or more types of sucrose fatty acid esters and polyglyceryl fatty acid esters.

The total amount formulated of the one or two or more types of sucrose fatty acid esters and polyglyceryl fatty acid esters usable in the present invention is preferably 0.1 to 10.0% by weight, more preferably 0.3 to 5.0% by weight, based upon the total weight of the composition. If the total amount is less than 0.1% by weight, the emulsifiability is liable not to be expressed, while even if the total amount is more than 10.0% by weight, a further improvement in the emulsion stability cannot be hoped.

As the silicone-based oil (c) usable in the present invention, dimethyl polysiloxane, methylphenyl polysiloxane, amino-modified silicone, fluorine-modified silicone, etc. may be mentioned.

The amount formulated of the silicone-based oil (c) is not particularly limited, but preferably the ratio of the silicone oil in the total oil is 10 to 100% by weight, more preferably 50 to 100% by weight. If the amount is less than 10% by weight, the characteristic silky feeling in use of silicone oil is liable to be lost.

Further, as the inorganic salt (d) used in the present invention, an alkali metal salt, alkali earth metal salt, aluminum salt, zinc salt, ammonium salt, etc. of hydrochloric acid, sulfuric acid, nitric acid, carbonic acid, phosphoric acid, etc. may be mentioned. As a preferable inorganic salt, sodium chloride, potassium chloride, magnesium chloride, calcium chloride, ammonium chloride, zinc chloride, ammonium chloride, and other chlorides; sodium sulfate, potassium sulfate, magnesium sulfate, aluminum sulfate, zinc sulfate, ammonium sulfate, and other sulfates; sodium nitrate, potassium nitrate, magnesium nitrate, calcium nitrate, aluminum nitrate, zinc nitrate, ammonium nitrate, and other nitrates; sodium carbonate, potassium carbonate, magnesium carbonate, calcium carbonate, and other carbonates; sodium phosphate, potassium phosphate, and other phosphates may be mentioned. Among these, sodium chloride, potassium chloride, magnesium chloride, calcium chloride, aluminum chloride, sodium sulfate, potassium sulfate, magnesium sulfate, and aluminum sulfate are particularly preferable.

Further, as the organic salt (d), sodium alginate, sodium asparagate, sodium citrate, sodium lactate, sodium ascorbate, sodium glutamate, etc. may be mentioned.

The total amount formulated of the inorganic salt and/or organic salt used in the present invention is not particularly limited, but preferably is 0.1 to 8.0% by weight, more preferably 0.5 to 5.0% by weight, based upon total weight of the composition. If the formulated amount is less than 0.1% by weight or more than 8.0% by weight, the stabilization of the emulsion cannot be stabilized.

The ratio of the components (a) to (c) and the component (d) is preferably, by weight ratio, (a) to (c):(d) = 4:1 to 20:1, in particular 4.3:1 to 10:1, in terms of useability and stability.

Further, the water-in-oil emulsion composition of the present invention may include a polyhydric alcohol or humectant to an extent not impairing the effects of the present invention so as to enhance the moisture retention effect.

For example, as a polyhydric alcohol, bivalent alcohols such as ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, octylene glycol; trivalent alcohols such as glycerol, trimethylol propane; 1,2,6-hexane triol; quadravalent alcohols such as pentaerylthritol pentavalent alcohols such as xylitol; hexavalent alcohols such as sorbitol, mannitol; polyhydric alcohol copolymers such as diethylene glycol, dipropylene glycol, triethylene glycol, polypropylene glycol, triglycerin, tetraglycerin, polyglycerin; and
bivalent alcohol alkyl ethers such as ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether; bivalent alcohol alkyl ethers such as diethylene glycol monomethyl ether, diethylene glycol monoethyl ether, diethylene glycol monobutyl ether, diethylene glycol dimethyl ether, diethylene glycol diethyl ether, diethylene glycol dibutyl ether, diethylene glycol methylethyl ether, triethylene glycol monomethyl ether, triethylene glycol monoethyl ether, triethylene glycol monoethyl ether, propylene glycol monomethyl ether, propylene glycol monoethyl ether, propylene glycol monobutyl ether, propylene glycol monoisopropyl ether, dipropylene glycol methyl ether, dipropylene glycol ethyl ether, dipropylene glycol butyl ether; bivalent alcohol ether esters such as, ethylene glycol monomethyl ether acetate, ethylene glycol monoethyl ether acetate, ethylene glycol monobutyl ether acetate, ethylene glycol monophenyl ether acetate, ethylene glycol disuccinate, ethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate; sugaralcohols such as xyl alcohol, monooleyl glyceryl ether, batyl alcohol, and other glyceryl monoalkyl ethers, sorbitol, maltitol, maltitrose, mannitol, sucrose, erythritol, glucose, fructose, starch sugar, maltose, xylitose, starch sugar reducing alcohol; glyceride, tetrahydrofurfuryl alcohol, POE tetrahydrofurfuryl alcohol, POP butyl ether, POP•POE butyl ether, methyl polyoxypropylene glyceryl ether, POP glyceryl ether, POP glyceryl ether phosphoric acid, POP•POE pentaerythritol ether, etc. may be mentioned.

As a humectant, chondroitin sulfate, hyaluronic acid, mucoitin-sulfuric acid, caronic acid, atelocollagen, cholestearyl-12-hydroxystearate, bile acid monosalt, dl-pyrrolidonecarboxylic acid monosalt, short-chain soluble collagen, hestnut rose extract, yarrow extract, etc. may be mentioned.

It is also possible to include an aqueous polymer to an extent not impairing the feeling in use, pH, etc. of the emulsion composition of the present invention. As a natural water-soluble polymer, for example, plant-based polymers such as gum arabic, gum tragacanth, galactan, guar gum, carob gum, gum karaya, carrageenan, pectin, agar, quince seed, algae colloid, starch (rice, corn, potato, wheat), glycyrrhizinic acid; microorganism-based polymers such as xanthane gum, dextran, succinoglucan, pullulan; animal-based polymers such as collagen, casein, albumin, gelatin; etc. may be mentioned.

As a semisynthesized water-soluble polymer, for example, starch-based polymers such as carboxymethyl starch, methylhydroxypropyl starch; cellulose-based polymers such as methylcellulose, nitrocellulose, ethylcellulose, methylhydroxypropylcellulose, hydroxyethylcellulose, sodium cellulose sulfate, hydroxypropylcellulose, sodium carboxymethylcellulose (CMC), crystalline cellulose, cellulose powder; alginic acid-based polymers, such as sodium alginate, alginic acid propylene glycol esters; etc. may be mentioned.

As a synthesized water-soluble polymer, for example, vinyl-based polymers such as polyvinyl alcohol, polyvinyl methyl ether-based polymers, polyvinylpyrrolidone, carboxyvinyl polymer (CARBOPOL 940, 941; BF Goodrich); polyoxyethylene-based polymers such as polyethylene glycol 20,000, polyethylene glycol 6,000, polyethylene glycol 4,000; copolymer-based polymers such as polyoxyethylene polyoxypropylene copolymers; acryl-based polymers such as sodium polyacrylate, polyethyl acrylate, polyacrylamide; polyethyleneimine; cationic polymers, etc. may be mentioned.

The emulsion composition of the present invention may also include various ingredients normally included in the field of cosmetic and pharmaceutical compositions. For example, water-soluble active substances such as vitamin B group, vitamin C and its derivatives, pantothenic acid and its derivatives, biotin, and other vitamins; buffer agents such as alginin, asparagic acid, citric acid, tartaric acid, lactic acid; chelating agents such as EDTA; preservatives, etc. and also ultraviolet absorbing agents various types of powders, various types of dyes, etc. may be mentioned. Further, it is also possible to include, in the range not impairing the effect of the present invention, an anionic surfactant, nonionic surfactant, cationic surfactant, or bipolar surfactant.

The water-in-oil emulsion composition of the present invention is used as, for example, skin cosmetics, hair cosmetics, skin external preparations, etc. in the fields of cosmetics, quasi-drugs, and pharmaceuticals. Since it has a superior feeling in use, it is preferably used as an emusion cosmetic composition.

### EXAMPLES

The present invention will now be explained in further detail using Examples and Comparative Examples, but the present invention is, of course, not limited to these Examples. Note that "%" all mean "% by weight".

### Examples 1 to 4 and Comparative Examples 1 to 4

Moisturizing creams of water-in-oil emulsion compositions were prepared by an ordinary method using the formulations of Table I and Table II and evaluated according to the following methods for feeling in use and stability. The results of the evaluation are also shown in Table I and Table II. It is understood from Table I and Table II that the water-in-oil type emulsion compositions of the present invention are excellent in stability and having superior feelings in use.

### (1) Evaluation of Feeling in Use

A panel of 10 women was used to evaluate the moisturizing creams for useability when applied for use based on the following evaluation criteria.
(i) Freshness
   ++: At least eight of 10 panelists respond cream feels fresh
   +: At least six panelists respond cream feels fresh
   ±: At least four panelists respond cream feels fresh
   -: Less than four panelists respond cream feels fresh
(ii) Sharpness
   ++: At least eight of 10 panelists respond cream feels sharp
   +: At least six panelists respond cream feels sharp
   ±: At least four panelists respond cream feels sharp
   -: Less than four panelists respond cream feels sharp

### (2) Evaluation of Stability

The creams were tested and evaluated for changes in appearance after being left standing at 0°C, room temperature, 37°C, and 50°C by the following criteria:
++: No abnormality
+: Slight partial separation of oil
±: Separation of oil
-: Separation

**Table I**

| Examples | 1 | 2 | 3 | 4 |
|---|---|---|---|---|
| Decamethylcyclopentasiloxane | 10.0 | 10.0 | 10.0 | 10.0 |
| Polydimethyl silicone | 5.0 | 5.0 | 5.0 | 5.0 |
| Polyether-modified silicone*1 | 1.5 | 0.5 | 1.5 | 1.5 |
| High molecular weight | 1.5 | 0.5 | 1.5 | 1.5 |
| polyether-modified silicone*2 | | | | |
| Purified water | Balance | Balance | Balance | Balance |
| Glycerol | 5.0 | 5.0 | 5.0 | 5.0 |
| Vitamin C derivative | 0.1 | 0.1 | 0.1 | 0.1 |
| Sodium L-glutamate | 2.0 | 2.0 | - | 0.5 |
| Sodium chloride | - | - | 1.0 | 0.5 |

| Useability | | | | |
|---|---|---|---|---|
| Freshness | + | ++ | + | + |
| Sharpness | + | ++ | + | + |

| Stability | | | | |
|---|---|---|---|---|
| 0°C | ++ | ++ | ++ | ++ |
| Room temperature | ++ | ++ | ++ | ++ |
| 37°C | ++ | ++ | ++ | ++ |
| 50°C | ++ | ++ | ++ | ++ |

**Table II**

| Examples | 1 | 2 | 3 | 4 |
|---|---|---|---|---|
| Decamethylcyclopentasiloxane | 10.0 | 30.0 | 10.0 | 10.0 |
| Polydimethyl silicone | 5.0 | 10.0 | 5.0 | 5.0 |
| High molecular weight | - | - | 3.0 | 1.0 |
| polyether-modified silicone*1 | | | | |
| Polyether-modified silicone*2 | 3.0 | - | - | 1.0 |
| Purified water | Balance | Balance | Balance | Balance |
| Glycerol | 5.0 | 5.0 | 5.0 | 5.0 |
| Ascorbic acid | 0.1 | 0.1 | 0.1 | 0.1 |
| Magnesium sulfate | 2.0 | 2.0 | 2.0 | - |

| Useability | | | | |
|---|---|---|---|---|
| Freshness | + | - | + | + |
| Sharpness | + | - | + | + |

| Stability | | | | |
|---|---|---|---|---|
| 0°C | ++ | No emulsion | ++ | ++ |
| Room temperature | + | No emulsion | + | + |
| 37°C | ± | No emulsion | ± | ± |
| 50°C | - | No emulsion | - | - |

| | | | | |
|---|---|---|---|---|
| *1: Number average molecular weight 55000 | | | | |
| *2: Number average molecular weight 6000 | | | | |

### Example 5: Moisturizing Cream

| Ingredient | % by weight |
|---|---|
| (1) Purified water | Balance |
| (2) Sodium chloride | 1.0 |
| (3) 1,3-butylene glycol | 5.0 |
| (4) Calcium chloride | 1.0 |
| (5) Hyaluronic acid | 0.1 |
| (6) Methyl paraben | q.s. |
| (7) Octamethylcyclopentasiloxane | 10.0 |
| (8) Methylphenyl polysiloxane | 4.0 |
| (9) High molecular weight polyether-modified silicone | 1.0 |
| (number average molecular weight 40000) | |
| (10) Polyether-modified silicone (number average | 1.0 |
| molecular weight 6000) | |
| (11) Liquid paraffin | 1.0 |
| (12) Vitamin E acetate | 1.0 |
| (13) Fragrance | q.s. |

### Process of Preparation

The ingredients (7) to (13) were mixed at room temperature to homogeneously disperse the oil phase. An aqueous phase obtained by adding (6) to (3) and dissolving them, then further adding (2), (4), and (5) was gradually added to the oil phase and homogeneously dispersed by a homodisperser, then the emulsion particles were graded to prepare a moisturizing cream as a water-in-oil type emulsion cosmetic composition. The moisturizing cream obtained had an excellent stability and superior useability.

### Example 6: Cosmetic Foundation

| Ingredient | % by weight |
|---|---|
| (1) Ion exchanged water | Balance |
| (2) 1,3-butylene glycol | 5.0 |
| (3) Sodium citrate | 1.0 |
| (4) Urea | 1.0 |
| (5) Albutin | 1.0 |
| (6) Ethanol | 5.0. |
| (7) High molecular weight polyether-modified silicone | 1.5 |
| (number average molecular weight 65000) | |
| (8) Polyether-modified silicone (number average | 1.5 |
| molecular weight 6000) | |
| (9) Octylmethoxy cinnamate | 2.0 |
| (10) Octamethylcyclopentasiloxane | 13.0 |
| (11) Particulate titanium oxide | 3.0 |
| (12) Ethyl paraben | q.s. |
| (13) Dibutylhydroxy toluene | q.s. |
| (14) Fragrance | q.s. |

### Process of Preparation

The ingredients (7) to (14) were dissolved by heating to homogeneously disperse the oil phase. An aqueous phase obtained by mixing (2) to (6) was gradually added to the oil phase and homogeneously dispersed by a homodisperser, then the emulsion particles were graded to prepare a cosmetic foundation as a water-in-oil type emulsion cosmetic. The cosmetic foundation obtained had an excellent stability, no stickiness of an ultraviolet absorber etc., and a superior useability.

### Example 7. Wipeoff Type Makeup Remover

| Ingredient | % by weight |
|---|---|
| (1) Purified water | Balance |
| (2) Potassium chloride | 1.0 |
| (3) 1,3-butylene glycol | 5.0 |
| (4) Trimethyl glycerin | 1.0 |
| (5) Methyl paraben | q.s. |
| (6) Ethanol | 5.0 |
| (7) POE-hydrogenated castor oil (HLB=18) | 1.0 |
| (8) Octamethylcyclopentasiloxane | 12.0 |
| (9) High molecular weight polyether-modified silicone (number average molecular weight 70000) | 1.0 |
| (10) Polyether-modified silicone (number average molecular weight 6000) | 1.0 |
| (11) Fragrance | q.s. |

### Process of Preparation

The ingredients (8) to (11) were mixed at room temperature to homogeneously disperse the oil phase. An aqueous phase obtained by adding (5) to (3) and dissolving them, then further adding (2), (4), (6), and (7) was gradually added to the oil phase and homogeneously dispersed by a homodisperser, then the emulsion particles were graded to prepare a moisturizing cream as a water-in-oil type emulsion cosmetic composition. The moisturizing cream obtained had an excellent stability and superior useability.

### Example 8: Body Massage Cream

| Ingredient | % by weight |
|---|---|
| (1) Ion exchanged water | Balance |
| (2) Dipropylene glycol | 3.0 |
| (3) Polyethylene glycol 1500 | 1.0 |
| (4) Vitamin C derivative | 0.5 |
| (5) Sodium lactate | 1.0 |
| (6) Phenoxyethanol | q.s. |
| (7) High molecular weight polyether-modified silicone | 0.5 |
| (number average molecular weight 80000) | |
| (8) Polyether-modified silicone (number average | 1.5 |
| molecular weight 6000) | |
| (9) Polydimethyl silicone | 5.0 |
| (10) Dimethylcyclopentasiloxane | 7.0 |
| (11) Dibutylhydroxy toluene | q.s. |
| (12) Fragrance | q.s. |
| (13) Polyethylene powder | 3.0 |

### Process of Preparation

The ingredients (7) to (12) were homogeneously dissolved, then (13) added and homogeneously dispersed. An aqueous phase obtained by mixing (2) to (6) was gradually added to the oil phase and homogeneously dispersed by a homodisperser, then the emulsion particles were graded to prepare a cosmetic foundation as a water-in-oil type emulsion cosmetic composition. The cosmetic foundation obtained had an excellent stability, no stickiness of the UV absorber etc., and superior useability.

### Examples 9 to 13 and Comparative Examples 5 to 9

Moisturizing creams of water-in-oil type emulsion compositions were prepared by an ordinary method by the formulations of Table III and Table IV and were evaluated for useability and stability according to the above methods. The results of evaluation are shown together in Table III and Table IV. From Table III and Table IV, it will be understood that the water-in-oil type emulsion compositions of the present invention have excellent stability and superior useability.

**Table III**

| Examples | 9 | 10 | 11 | 12 | 13 |
|---|---|---|---|---|---|
| Decamethylcyclopenta- | 10.0 | 10.0 | 15.0 | 5.0 | 10.0 |
| siloxane | | | | | |
| Cetyl octanate | 5.0 | 5.0 | 5.0 | 5.0 | 5.0 |
| Sucrose palmitic ester | 3.0 | 1.0 | - | 2.0 | - |
| Sucrose isostearic acid | | | | | |
| ester | - | - | - | - | 1.0 |
| Decaglyceryl oleic acid | | | | | |
| ester | - | 1.0 | 1.5 | 2.0 | 1.0 |
| High molecular weight | | | | | |
| polyether-modified | 3.0 | 1.0 | 1.5 | 0.1 | 1.0 |
| silicone*3 | | | | | |
| Purified water | Balance | Balance | Balance | Balance | Balance |
| Glycerol | 5.0 | 5.0 | 5.0 | 5.0 | 5.0 |
| Vitamin C derivative | 0.1 | 0.1 | 0.1 | 0.1 | 0.1 |
| Sodium L-glutamate | 2.0 | 2.0 | 2.0 | 2.0 | 2.0 |

| Useability | | | | | |
|---|---|---|---|---|---|
| Freshness | + | ++ | + | + | ++ |
| Sharpness | + | ++ | ++ | + | ++ |

| Stability | | | | | |
|---|---|---|---|---|---|
| 0°C | ++ | ++ | ++ | ++ | ++ |
| Room temperature | ++ | ++ | ++ | ++ | ++ |
| 37°C | ++ | ++ | ++ | ++ | ++ |
| 50°C | ++ | ++ | ++ | + | ++ |

**Table IV**

| Comparative Examples | 5 | 6 | 7 | 8 | 9 |
|---|---|---|---|---|---|
| Decamethylcyclopenta- | 10.0 | 10.0 | 10.0 | 10.0 | 2.0 |
| siloxane | | | | | |
| Cetyl octanate | 5.0 | 5.0 | 5.0 | 5.0 | 2.0 |
| Sucrose palmitic acid | - | 3.0 | - | 1.5 | 0.03 |
| ester | | | | | |
| Octaglyceryl eruca acid | - | - | 3.0 | - | 0.02 |
| ester | | | | | |
| High molecular weight | 3.0 | - | - | 1.5 | 0.05 |
| polyether-modified | | | | | |
| silicone*3 | | | | | |
| Purified water | Balance | Balance | Balance | Balance | Balance |
| Glycerol | 5.0 | 5.0 | 5.0 | 5.0 | 5.0 |
| Ascorbic acid | 0.1 | 0.1 | 0.1 | 0.1 | 0.1 |
| Magnesium sulfate | 2.0 | 2.0 | - | - | 2.0 |

| Useability | | | | | |
|---|---|---|---|---|---|
| Freshness | ++ | ++ | ++ | ++ | None |
| Sharpness | ++ | ++ | ++ | ++ | None |

| Stability | | | | | |
|---|---|---|---|---|---|
| 0°C | ++ | ++ | ++ | ++ | No emulsion |
| Room temperature | + | + | + | + | No emulsion |
| 37°C | ± | ± | ± | ± | No emulsion |
| 50°C | - | - | - | - | No emulsion |

| | | | | | |
|---|---|---|---|---|---|
| *3: Number average molecular weight 55000 | | | | | |

### Example 14: Moisturizing Cream

| Ingredient | % by weight |
|---|---|
| (1) Purified water | Balance |
| (2) Table salt | 1.0 |
| (3) 1,3-butylene glycol | 5.0 |
| (4) Trehalose | 3.0 |
| (5) Hyaluronic acid | 0.1 |
| (6) Methyl paraben | q.s. |
| (7) Octamethylcyclopentasiloxane | 10.0 |
| (8) Glyceryl trioctanate | 4.0 |
| (9) Liquid paraffin | 2.0 |
| (10) High molecular weight polyether-modified | 2.0 |
| silicone (number average molecular weight 40000) | |
| (11) Sucrose oleaic acid ester | 2.0 |
| (12) Vitamin E acetate | 1.0 |
| (13) Fragrance | q.s. |

### Process of Preparation

The ingredients (7) to (13) were mixed at room temperature to homogeneously disperse the oil phase. An aqueous phase obtained by adding (6) to (3) and dissolving them, then further adding (2), (4), and (5) was gradually added to the oil phase and homogeneously dispersed by a homodisperser, then the emulsion particles were graded to prepare a moisturizing cream as a water-in-oil type emulsion cosmetic composition. The moisturizing cream obtained had an excellent stability and superior useability.

### Example 15: Cosmetic Foundation

| Ingredient | % by weight |
|---|---|
| (1) Ion exchanged water | Balance |
| (2) 1,3-butylene glycol | 5.0 |
| (3) Sorbitol | 1.0 |
| (4) Fructose | 1.0 |
| (5) Albutin | 1.0 |
| (6) Ethanol | 5.0. |
| (7) Sucrose eruca acid ester | 1.5 |
| (8) High molecular weight polyether-modified silicone | 1.5 |
| (number average molecular weight 65000) | |
| (9) Octylmethoxy cinnamate | 5.0 |
| (10) Particulate titanium oxide | 3.0 |
| (11) Ethyl paraben | q.s. |
| (12) Dibutylhydroxy toluene | q.s. |
| (13) Fragrance | q.s. |

### Process of Preparation

The ingredients (7) to (13) were dissolved by heating to homogeneously disperse the oil phase. An aqueous phase obtained by mixing (2) to (6) was gradually added to the oil phase and homogeneously dispersed by a homodisperser, then the emulsion particles were graded to prepare a cosmetic foundation as a water-in-oil type emulsion cosmetic composition. The cosmetic foundation obtained had an excellent stability, no stickiness of an ultraviolet absorber etc., and a superior useability.

### Example 16: Wipeoff Type Makeup Remover

| Ingredient | % by weight |
|---|---|
| (1) Purified water | Balance |
| (2) Potassium chloride | 1.0 |
| (3) 1,3-butylene glycol | 5.0 |
| (4) Trimethyl glycerin | 1.0 |
| (5) POE-hydrogenated castor oil (HLB=18) | q.s. |
| (6) Octamethylcyclopentasiloxane | 12.0 |
| (7) Liquid paraffin | 1.0 |
| (8) High molecular weight polyether-modified silicone | 1.0 |
| (number average molecular weight 70000) | |
| (9) Sucrose oleaic acid ester | 1.0 |
| (10) Decaglyceryl eruca acid ester | 2.0 |
| (11) Fragrance | q.s. |

### Process of Preparation

The ingredients (7) to (13) were mixed at room temperature to homogeneously disperse the oil phase. An aqueous phase obtained by adding (6) to (3) and dissolving them, then further adding (2), (4), and (5) was gradually added to the oil phase and homogeneously dispersed by a homodisperser, then the emulsion particles were graded to prepare a moisturizing cream as a water-in-oil type emulsion cosmetic composition. The moisturizing cream obtained had an excellent stability and superior useability.

### Example 17. Body Massage Cream

| Ingredient | %by weight |
|---|---|
| (1) Ion exchanged water | Balance |
| (2) Dipropylene glycol | 3.0 |
| (3) Polyethylene glycol 1500 | 1.0 |
| (4) Vitamin C derivative | 0.5 |
| (5) Ethanol | 7.0 |
| (6) Tetraglyceryl isostearic acid ester | 2.0 |
| (7) High molecular weight polyether-modified | 1.5 |
| silicone (number average molecular weight 80000) | |
| (8) Dimeticon | 5.0 |
| (9) Octamethylcyclopentasiloxane | 7.0 |
| (10) Polyethylene powder | 3.0 |
| (11) Phenoxyethanol | q.s. |
| (12) Dimethylcyclopentasiloxane | q.s. |
| (13) Fragrance | q.s. |

### Process of Preparation

The ingredients (7) to (13) were dissolved by heating to homogeneously dissolve the oil phase. An aqueous phase obtained by mixing (2) to (6) was gradually added to the oil phase and homogeneously dispersed by a homodisperser, then the emulsion particles were graded to prepare a cosmetic foundation as a water-in-oil type emulsion cosmetic composition. The cosmetic foundation obtained had an excellent stability, no stickiness of the UV absorber etc., and superior useability.

### INDUSTRIAL APPLICABILITY

As explained above, according to the present invention, it is possible to provide a high internal aqueous phase water-in-oil type emulsion composition and a cosmetic emulsion having a good stability and having a fresh, sharp feeling in use. Therefore, this is expected to be useful in cosmetic compositions and other quasi-drugs, pharmaceuticals, etc.

## Claims

1. A water-in-oil type emulsion composition comprising:
(a) at least one high molecular weight polyether-modified silicone having a number-average molecular weight of at least 30,000 and having the formula (I): 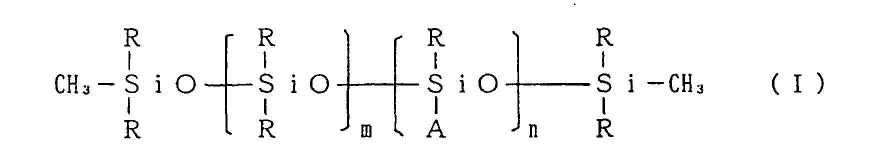
wherein A is a polyoxyalkylene group having the formula: -C₃H₆O(C₂H₄O)ₐ(C₃H₆O)_{b}R', wherein R' is a group selected from the group consisting of a hydrogen atom, an acyl group, and a C₁ to C₄ alkyl group, a is an integer of 5 to 50 and b is an integer of 5 to 50, R is a methyl group or a phenyl group, m is an integer of 50 to 1000, and n is an integer of 1 to 40;
(b) at least one polyether-modified silicone having a number-average molecular weight of 10,000 or less and having the formula (II): 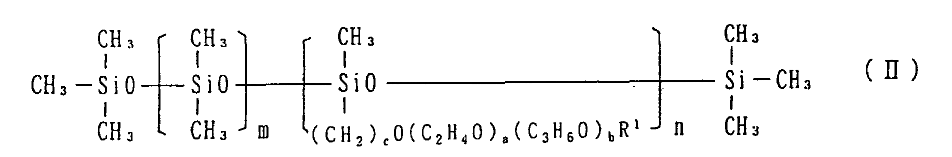
wherein R¹ indicates a hydrogen atom or a C₁ to C₅ alkyl group, m is on average a number of 1 to 150, n is on average a number of 1 to 50, a and b are independently, on average, numbers of 0 to 35, and c is a number of 1 to 5;
(c) a silicone-based oil; and
(d) at least one salt selected from the group consisting of inorganic salts and organic salts.

2. A water-in-oil emulsion composition as claimed in claim 1, wherein the ratio of composition of the components (a) to (c) and component (d) is, by a weight ratio, 4:1 to 20:1.

3. A water-in-oil emulsion composition as claimed in claim 1, wherein the content of the component (d) is 0.01 to 10.0% by weight, based upon the total weight of the composition.

4. A water-in-oil type the emulsion composition comprising:
(a) at least one high molecular weight polyether-modified silicone having a molecular weight of at least 30,000 and having the formula (I): 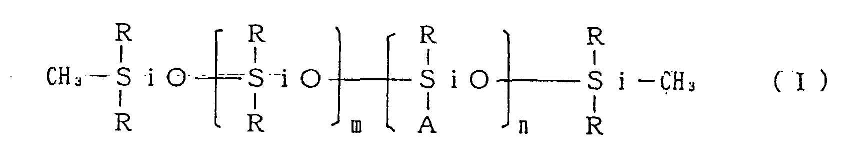
wherein A is a polyoxyalkylene group having the formula-C₃H₆O(C₂H₄O)ₐ(C₃H₆O)_{b}R, wherein R' is a group selected from the group consisting of a hydrogen atom, an acyl group, and a C₁ to C₄ alkyl group, a is an integer of 5 to 50 and b is an integer of 5 to 50, R is a methyl group or a phenyl group, m is an integer of 50 to 1000, and n is an integer of 1 to 40;
(b) at least one fatty acid ester selected from the group consisting of sucrose fatty acid esters and polyglyceryl fatty acid esters, which are liquid at ordinary temperature and have an HLB value of 4 or less;
(c) a silicone-based oil; and
(d) at least one salt selected from the group consisting of inorganic salts and organic salts.

5. A water-in-oil type emulsion composition as claimed in claim 4, wherein at least 20% by weight of the component fatty acid of the component (b) is a C₈ to C₁₈ saturated fatty acid ester or a C₁₆ to C₂₂ unsaturated fatty acid and at least 95% by weight of the sucrose fatty acid ester in the component (b) is a polyester.

6. A water-in-oil type emulsion composition as claimed in claim 4, wherein a ratio of the components (a) to (c) and the component (d) is, by a weight ratio, 4:1 to 20:1.

7. A water-in-oil type the emulsion composition as claimed in claim 4, wherein the content of the component (d) is 0.01 to 10.0% by weight, based upon the total weight of the composition.

8. A water-in-oil type emulsion type composition as claimed in any one of claims 1 to 7, which is an emulsion cosmetic composition.
